# EUROPÄISCHE PATENTANMELDUNG

(11) **EP 4 548 869 A1**
(43) Veröffentlichungstag der Anmeldung: **07.05.2025**
(21) Anmeldenummer: 24206687.6
(22) Anmeldetag: 15.10.2024
(51) Int. Cl.: A61B 18/14

(54) **BIPOLARE ELEKTRODE FÜR EIN RESEKTOSKOP SOWIE RESEKTOSKOP MIT EINER SOLCHEN BIPOLAREN ELEKTRODE**

(30) Priorität: 30.10.2023 DE 102023129912
(71) Anmelder: Henke-Sass, Wolf GmbH, 78532 Tuttlingen (DE)
(72) Erfinder: PFAFF, Armin, 78655 Dunningen (DE); RAPP, Stefan, 78166 Donaueschingen (DE)
(74) Vertreter: Patentanwälte Geyer, Fehners & Partner mbB

(57) **Zusammenfassung**

Es wird eine Bipolare Elektrode für ein Resektoskop, bereitgestellt,
wobei die bipolare Elektrode (20) einen proximalen Kontaktabschnitt (25), einen daran anschließenden Mittelabschnitt (26) und einen daran anschließenden distalen Endabschnitt (27) aufweist,
wobei die bipolare Elektrode (20) eine Aktivelektrode (34) und eine Neutralelektrode (38) aufweist,
wobei die Neutralelektrode (38) im Mittelabschnitt (26) hohlzylinderförmig ausgebildet ist und die Aktivelektrode (34) koaxial umgibt,
wobei der proximale Kontaktabschnitt (25) einen ersten Kontakt (30) der Aktivelektrode (34) und einen zweiten Kontakt (36) der Neutralelektrode (38) aufweist.
wobei die Neutralelektrode (38) vom proximalen Kontaktabschnitt (25) bis zum distalen Endabschnitt (27) mit einer ersten Isolierung (39) umgeben ist,
und wobei am distalen Endabschnitt (27) sowohl ein erster Endbereich der Aktivelektrode (34) als auch ein zweiter Endbereich der Neutralelektrode (38) freiliegt, so dass eine über die Kontakte (30, 36) angelegte elektrische Spannung unmittelbar zwischen den beiden Endbereichen anliegt.

## Beschreibung

Die vorliegende Erfindung betrifft eine bipolare Elektrode für ein Resektoskop sowie ein Resektoskop mit einer solchen bipolaren Elektrode.

Bekannte Resektoskope mit einer bipolaren Elektrode sind so ausgebildet, dass die Neutralelektrode der bipolaren Elektrode über das Resektoskop selbst oder eine separate Kontaktierung am Patienten erfolgt. Dadurch kann es schwierig werden, das gewünschte Plasma am distalen Ende der bipolaren Elektrode für die Behandlung zuverlässig zu zünden.

Ausgehend hiervon ist es daher Aufgabe der Erfindung, eine verbesserte bipolare Elektrode für ein Resektoskop und ein Resektoskop mit einer solchen bipolaren Elektrode bereitzustellen.

Die Erfindung ist in den unabhängigen Ansprüchen 1 und 13 definiert. Vorteilhafte Ausgestaltungen sind in den abhängigen Ansprüchen angegeben.

Die erfindungsgemäße bipolare Elektrode für ein Resektoskop kann einen proximalen Kontaktabschnitt, einen daran anschließenden Mittelabschnitt und einen daran anschließenden distalen Endabschnitt aufweisen, wobei die bipolare Elektrode eine Aktivelektrode und eine Neutralelektrode umfasst. Die Neutralelektrode kann im Mittelabschnitt hohlzylinderförmig ausgebildet sein und die Aktivelektrode koaxial umgeben. Der proximale Kontaktabschnitt kann einen ersten Kontakt der Aktivelektrode und einen zweiten Kontakt der Neutralelektrode aufweisen, wobei die Neutralelektrode vom proximalen Kontaktabschnitt bis zum distalen Endabschnitt mit einer ersten Isolierung umgeben ist. Am distalen Endabschnitt liegen ein erster Endbereich der Aktivelektrode und ein zweiter Endbereich der Neutralelektrode frei, so dass eine über die Kontakte angelegte elektrische Spannung unmittelbar zwischen den beiden Endbereichen anliegt.

Damit kann sichergestellt werden, dass das gewünschte Plasma bei der Behandlung zuverlässig gezündet wird.

Die Aktivelektrode kann im Mittelabschnitt einen Kupferleiter aufweisen. Mit einem solchen Kupferleiter können die notwendigen hohen Ströme sicher geleitet werden, ohne dass es zu Temperaturproblemen während der Behandlung kommt.

Die Aktivelektrode kann im Mittelabschnitt einen hohlzylinderförmigen Leiter aufweisen. Im hohlzylinderförmigen Leiter kann ein leitfähiger Draht angeordnet sein. Insbesondere kann der hohlzylinderförmige Leiter aus Kupfer hergestellt sein.

Ferner kann die Aktivelektrode im Mittelabschnitt Vollmaterial-Leiter aufweisen. Hierunter wird insbesondere verstanden, dass der Leiter nicht hohl ist.

Ferner kann die Aktivelektrode im Bereich des proximalen Kontaktabschnitts ein Element zur mechanischen Verstärkung aufweisen. Insbesondere kann das Element zur mechanischen Verstärkung zylinderförmig ausgebildet und in dem hohlzylinderförmigen Leiter der Aktivelektrode eingesetzt sein. Das Element zur mechanischen Verstärkung kann aus Wolfram, Edelstahl, Federstahl, etc. hergestellt sein. Ferner kann zwischen der Aktivelektrode und der Neutralelektrode eine zweite Isolierung vorgesehen sein.

Des Weiteren kann der distale Endabschnitt gabelförmig mit einem ersten und einem zweiten Gabelarm ausgebildet sein, wobei der freiliegende erste Endbereich der Aktivelektrode zwischen den beiden Gabelarmen liegt.

Jeder Gabelarm kann eine dritte Isolierung aufweisen, die zwischen der Aktivelektrode und der Neutralelektrode angeordnet ist und mit der zweiten Isolierung überlappt.

Der freiliegende erste Endbereich der Aktivelektrode kann als Wolframdraht ausgebildet sein. Es ist jedoch auch möglich, dass der freiliegende erste Endbereich der Aktivelektrode aus Edelstahl gebildet ist.

Die Neutralelektrode kann im Mittelbereich bzw. im Mittelabschnitt einen hohlzylinderförmigen Leiter aufweisen. Der Leiter kann insbesondere aus Edelstahl hergestellt sein.

Der erste Kontakt der Aktivelektrode und/oder der zweite Kontakt der Neutralelektrode kann eine metallische Hülse aufweisen. Die metallische Hülse kann aus einem metallischen Leiter, wie z.B. Edelstahl hergestellt sein.

Der erste Kontakt der Aktivelektrode und/oder der zweite Kontakt der Neutralelektrode kann eine metallische Beschichtung aufweisen. Als metallische Beschichtung kann z.B. Gold, Silber, Aluminium oder Zink verwendet werden. Die metallische Beschichtung kann einen besseren elektrischen Kontakt und/oder eine verbesserte Korrosionsbeständigkeit bewirken.

Der zweite Kontakt der Neutralelektrode kann so ausgebildet sein, dass der zweite Kontakt in Eingriff mit einer Rastverbindung gebracht werden kann.

Der proximale Kontaktabschnitt der bipolaren Elektrode kann einen Außendurchmesser im Bereich von 0,8 - 1,2 mm und insbesondere einen Außendurchmesser von 1,0 mm aufweisen.

Der Außendurchmesser des Mittelabschnitts der bipolaren Elektrode kann im Bereich von 1,6 - 2,0 mm liegen und kann insbesondere 1,8 mm betragen.

Die Länge der bipolaren Elektrode kann im Bereich von 200 - 400 mm, insbesondere im Bereich von 250 - 350 mm und ferner insbesondere im Bereich von 280 - 300 mm liegen.

Jede der ersten, zweiten und dritten Isolierung kann als Schrumpfschlauch ausgebildet sein. Insbesondere kann ein Fluorpolymer-Schrumpfschlauch, z.B. ein PTFE-Schrumpfschlauch (Polytetrafluorethylen- Schrumpfschlauch) oder ein PVDF-Schrumpfschlauch (Polyvinylidenfluorid-Schrumpfschlauch) verwendet werden.

Die erfindungsgemäße bipolare Elektrode kann aufgrund ihres Aufbaus somit als koaxiale bipolare Elektrode bezeichnet werden, da die Neutralelektrode im Mittelabschnitt koaxial zur Aktivelektrode verläuft. Ferner werden beide Elektroden (Neutralelektrode und Aktivelektrode) vom proximalen Kontaktabschnitt bis zum distalen Ende geführt, so dass im Betrieb der Stromfluss zwischen den beiden freigelegten Endbereichen erfolgen kann. Daher muss in vorteilhafterweise bei der bestimmungsgemäßen Verwendung der erfindungsgemäßen bipolaren Elektrode kein Strom über den Patienten zu- bzw. abgeführt werden. Es liegt die volle Potenzialdifferenz (bzw. Spannung), die am proximalen Kontaktabschnitt der bipolaren Elektrode angelegt wird, an den beiden Endbereichen und somit am distalen Ende der bipolaren Elektrode an. Dadurch kann ein gutes Zündverhalten für das gewünschte Plasma z.B. in einer NaCl-Lösung sichergestellt werden.

Die bipolare Elektrode ist insbesondere für eine Spannung von 90 Volt - 4000 Volt (insbesondere 90 Volt - 1500 Volt oder 200 Volt - 1500 Volt) bei einer Frequenz von 300 kHz bis 4 MHz ausgelegt.

Der erste Endbereich der Aktivelektrode kann als Schlinge, Kegel, Messer, Walze, Rolle, etc. ausgebildet sein. Damit ist dann, wenn über die Kontakte eine entsprechende elektrische Spannung angelegt wird, ein Schneiden und/oder Koagulieren des entsprechenden Gewebes im Bereich des distalen Endes der bipolaren Elektrode möglich.

Ferner wird ein Resektoskop mit einer erfindungsgemäßen bipolaren Elektrode bereitgestellt.

Das Resektoskop kann einen Führungsblock aufweisen. In dem Führungsblock kann ein Rastelement vorgesehen sein, das im eingesetzten Zustand der bipolaren Elektrode eine Rastverbindung mit dem zweiten Kontakt der Neutralelektrode bewirkt. Alternativ kann das Rastelement im eingesetzten Zustand der bipolaren Elektrode eine Rastverbindung mit dem ersten Kontakt der Aktivelektrode bewirken. Der erste bzw. zweite Kontakt ist bevorzugt so ausgebildet, dass die gewünschte Rastverbindung (bevorzugt mittels Formschluss) vorliegt. Die Rastverbindung kann so ausgestaltet sein, dass die bipolare Elektrode im Führungsblock mechanisch fixiert ist und eine etwaige Verschiebung des Führungsblocks auch die bipolare Elektrode bewegt.

Der Führungsblock kann so ausgebildet sein, dass die bipolare Elektrode lösbar fixiert ist.

Der Führungsblock kann in Längsrichtung des Resektoskops verschiebbar sein.

Des Weiteren kann ein erster Kontaktabschnitt für den ersten Kontakt der Aktivelektrode und ein zweiter Kontaktabschnitt für den zweiten Kontakt der Neutralelektrode im Führungsblock vorgesehen sein, wobei zwischen beiden Kontaktabschnitten eine Dichtung angeordnet ist, die im eingesetzten Zustand der bipolaren Elektrode verhindert, dass Flüssigkeit vom ersten zum zweiten Kontakt (oder umgekehrt) gelangen kann.

Der erste und/oder zweite Kontaktabschnitt kann aus einem Metall, wie z.B. Edelstahl gebildet sein. Ferner kann der erste und/oder zweite Kontaktabschnitt eine metallische Beschichtung aufweisen. Als metallische Beschichtung kann z.B. Gold, Silber, Aluminium oder Zink verwendet werden. Die metallische Beschichtung kann einen besseren elektrischen Kontakt und/oder eine verbesserte Korrosionsbeständigkeit bewirken.

Das Resektoskop kann weitere, dem Fachmann bekannte Elemente aufweisen.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbeispiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine schematische perspektivische Darstellung einer Ausführungsform des erfindungsgemäßen Resektoskops;
- Fig. 2: eine Seitenansicht der erfindungsgemäßen bipolaren Elektrode 20;
- Fig. 3: eine Ansicht auf den distalen Endabschnitt 27 der bipolaren Elektrode 20;
- Fig. 4: eine vergrößerte Ansicht des Details B von Fig. 2 in einem Schnitt A-A gemäß Fig. 3;
- Fig. 5: eine Schnittansicht entlang der Schnittlinie C-C gemäß Fig. 3 durch den Führungsblock 4 mit eingesetzter bipolarer Elektrode 20;
- Fig. 6: eine Schnittansicht entlang der Schnittlinie A-A gemäß Fig. 3 durch den Führungsblock 4 mit eingesetzter bipolarer Elektrode 20;
- Fig. 7: eine Aufsicht des distalen Endabschnitts 27 der bipolaren Elektrode 20;
- Fig. 8: eine Schnittansicht entlang der Schnittlinie C-C gemäß Fig. 3 des Bereiches E in Fig. 7;
- Fig. 9: eine vergrößerte Ansicht des Details F von Fig. 8;
- Fig. 10: eine vergrößerte Ansicht des Details G von Fig. 8, und
- Fig. 11: eine Schnittansicht entlang der Schnittlinie D-D gemäß Fig. 3 des Gabelrohrs 59.

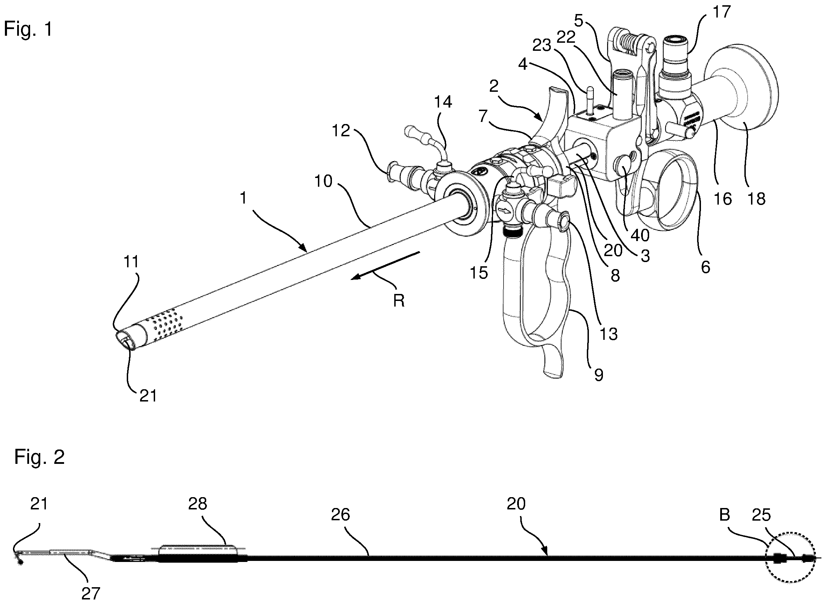

Bei der in Fig. 1 gezeigten Ausführungsform umfasst das erfindungsgemäße Resektoskop 1 ein Arbeitselement 2 mit einem Optikrohr 3, auf dem ein Führungsblock 4 in Längsrichtung R verschiebbar gelagert ist. Der Führungsblock 4 ist mittels eines Federelementes 5 in der in Fig. 1 gezeigten ersten Position gehalten. An dem Federelement 5 ist ein Daumenring 6 ausgebildet. Ferner weist das Arbeitselement 2 einen vom Führungsblock 4 in seiner ersten Position in Längsrichtung R beabstandeten vorderen Abschnitt 7 auf, der einen Anschlag 8 sowie einen Fingergriff 9 umfasst. Der vordere Abschnitt 7 ist fest mit dem Optikrohr 2 in der Weise verbunden, dass keine Bewegung in Längsrichtung R möglich ist.

Ferner umfasst das Resektoskop 1 einen Spülschaft 10, der an einem vom Führungsblock 4 wegweisenden Ende des vorderen Abschnitts 7 lösbar befestigt ist und in dem das Optikrohr 3 bis zu einem distalen Ende 11 des Spülschafts 10 läuft und somit vom Spülschaft 10 umgeben ist. Der Spülschaft 10 weist einen Zuflussanschluss 12 und einen Abflussanschluss 13 auf, die jeweils mit einem Ventil 14, 15 absperrbar sind.

An einem vom distalen Ende 11 des Spülschafts 10 wegweisenden Ende des Arbeitselementes 2 ist eine Beobachtungsoptik 16 lösbar verbunden, deren in Fig. 1 nicht gezeigte Endoskopbeobachtungsoptik sich durch das Optikrohr 3 bis zum distalen Ende 11 in bekannter Weise bei Resektoskopen erstreckt. In Fig. 1 sind ein Lichtleiteranschluss 17 sowie ein Einblick 18 gezeigt.

Des Weiteren umfasst das Resektoskop 1 eine erfindungsgemäße bipolare Elektrode 20, die im Führungsblock 4 lösbar fixiert ist, wie nachfolgend noch im Detail beschrieben wird. Die bipolare Elektrode 20 erstreckt sich durch den Spülschaft 10 bis zum distalen Ende 11 und steht in der in Fig. 1 gezeigten Position des Führungsblocks 4 nicht über das distale Ende 11 hinaus. Ein distales Ende 21 der Elektrode 20 ist somit noch innerhalb des Spülschaftes 10. Wenn jedoch durch einen Bediener der Führungsblock 4 in Richtung zum Anschlag 8 des vorderen Abschnitts 7 bewegt wird, wird dadurch auch die im Führungsblock 4 fixierte bipolare Elektrode 20 in dieser Richtung und somit in Längsrichtung R bewegt, wodurch das distale Ende 21 der bipolaren Elektrode 20 über das distale Ende 11 des Spülschafts 10 hinaus bewegt wird.

Es ist jedoch auch möglich, dass bei der in Fig. 1 gezeigten Position des Führungsblocks 4 das distale Ende 21 der Elektrode 20 über das distale Ende 11 des Spülschaft 10 hinaussteht. Wenn dann durch einen Bediener der Führungsblock 4 in Richtung zum Anschlag 8 des vorderen Abschnitts 7 bewegt wird, wird dadurch auch die im Führungsblock 4 fixierte bipolare Elektrode 20 in dieser Richtung und somit in Längsrichtung R bewegt, wodurch das distale Ende 21 der bipolaren Elektrode 20 weiter über das distale Ende 11 des Spülschafts 10 hinaus bewegt wird.

Der Führungsblock 4 weist einen ersten elektrischen Anschluss 22 für eine Aktivelektrode 34 der bipolaren Elektrode 20 und einen zweiten elektrischen Anschluss 23 für eine Neutralelektrode 38 der bipolaren Elektrode 20 auf.

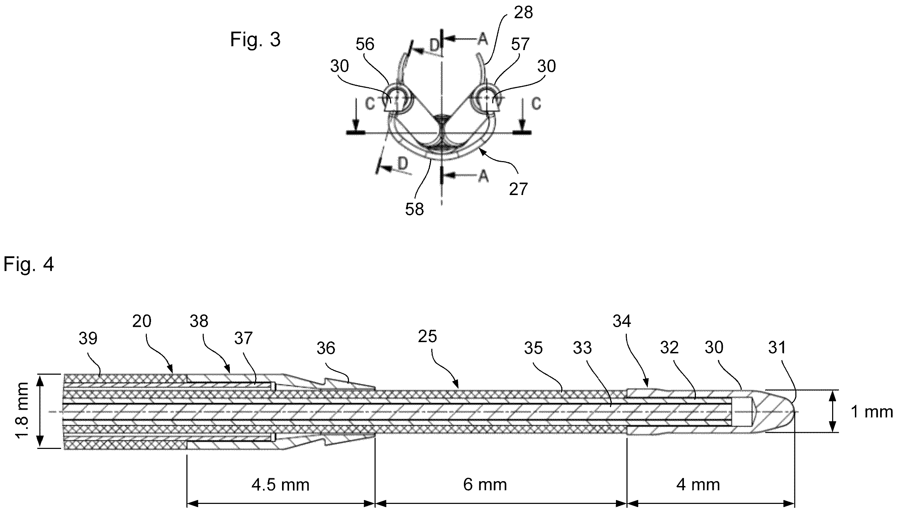

Wie am besten in Fig. 2 zu erkennen ist, umfasst die bipolare Elektrode 20 einen proximalen Kontaktabschnitt 25, einen daran anschließenden Mittelabschnitt 26 und einen daran anschließenden distalen Endabschnitt 27. An dem Mittelabschnitt 26 ist eine Führung 28 ausgebildet, die am Optikrohr 3 anliegt und die Bewegung der bipolaren Elektrode 20 in Längsrichtung R unterstützt. In Fig. 3 ist eine Ansicht auf den distalen Endabschnitt 27 der bipolaren Elektrode 20 zur Erläuterung der nachfolgenden Schnittansichten dargestellt. So ist der proximale Kontaktabschnitt 25 (Detail B in Fig. 2) im Schnitt A-A in Fig. 4 vergrößert dargestellt.

Am proximalen Ende des Kontaktabschnitts 25 ist eine Endhülse 30 mit einem abgerundeten proximalen Ende 31 ausgebildet, wobei die Endhülse 30 hier aus Edelstahl hergestellt ist und mit einem in die Endhülse 30 hineinstehenden Innenrohr 32, das bevorzugt hohlzylinderförmig ausgebildet ist, verpresst ist. Die Endhülse 30 bildet einen ersten Kontakt 30 der Aktivelektrode 34. Das Innenrohr 32 ist seinerseits mit einer zylinderförmigen Verstärkung 33 versehen, die im Innenrohr 32 an der inneren Wandung des Innenrohrs 32 anliegt. Diese Verstärkung 33 erstreckt sich mindestens über den gesamten proximalen Kontaktabschnitt 25. Als Material für die Verstärkung kann beispielsweise Wolfram, Federstahl, Draht, Edelstahl, etc. verwendet werden. Die Endhülse 30 sowie das Innenrohr 32, das bevorzugt aus Kupfer hergestellt ist, sind Teil der Aktivelektrode 34 der bipolaren Elektrode 20. In Längsrichtung R anschließend an die Endhülse 30 ist eine Isolierung 35 auf dem Innenrohr 32 ausgebildet. Die Isolierung 35 ist bevorzugt hohlzylinderförmig und kann beispielsweise als Schrumpfschlauch (z.B. PTFE) ausgebildet sein.

Auf der Isolierung 35 ist beabstandet in Längsrichtung R von der Endhülse 30 eine Rasthülse 36 angeordnet, die mit einem ebenfalls auf der Isolierung 35 angeordneten Hauptrohr 37 verbunden ist. Die Rasthülse 36 bildet einen zweiten Kontakt 36 der Neutralelektrode 38. Die Rasthülse 36 und das Hauptrohr 37 können beispielsweise jeweils aus Edelstahl hergestellt sein. Die Rasthülse 36 kann mit dem Hauptrohr 37 verschweißt sein. Dazu kann beispielsweise Laserschweißen eingesetzt werden. Die Rasthülse 36 sowie das Hauptrohr 37 sind Teile der Neutralelektrode 38 der bipolaren Elektrode 20. In Längsrichtung R anschließend an die Rasthülse 36 ist auf dem Hauptrohr 37 eine Isolierung 39, die nachfolgend auch als erste Isolierung 39 bezeichnet wird, ausgebildet. Ferner wird die Isolierung 35 zwischen Innenrohr 32 und Rasthülse 36 bzw. Hauptrohr 37 nachfolgend auch als zweite Isolierung 35 bezeichnet. Die erste Isolierung 39 kann in gleicher Weise wie die zweite Isolierung 35 als Schrumpfschlauch und insbesondere als PTFE-Schlauch ausgebildet sein. Es ist jedoch auch mögliche, dass die erste Isolierung 39 als PVDF-Schrumpfschlauch ausgebildet ist.

Die erste und die zweite Isolierung 39, 35 laufen vom Kontaktabschnitt 25 über den Mittelabschnitt 26 bis zum distalen Endabschnitt 27. Dabei dient die zweite Isolierung 35 zur Isolierung zwischen Aktivelektrode 34 und Neutralelektrode 38 und dient die erste Isolierung 39 zur Isolierung der Neutralelektrode 38 gegenüber der Umgebung.

Wie in Fig. 4 gut zu erkennen ist, dient die Verstärkung 33 insbesondere im Bereich zwischen Endhülse 30 und Rasthülse 36 zu einer mechanischen Stabilisierung des Kontaktabschnitts 25, da in diesem Bereich sonst nur das hohle Innenrohr 32 für die Stabilisierung sorgen müsste, was aufgrund des geringen Außendurchmessers von ca. 1 mm im Bereich der Endhülse 30 schwierig wäre.

Bei der hier beschriebenen Ausführungsform erstreckt sich die Endhülse 30 in Längsrichtung R über eine Strecke von 4 mm. Des Weiteren beträgt der Abstand zwischen Endhülse 30 und Rasthülse 36 6 mm und erstreckt sich die Rasthülse 36 in Längsrichtung R über eine Strecke von 4,5 mm, so dass der gesamte Kontaktabschnitt 25 eine Länge von 14,5 mm aufweist. Die Verstärkung 33 ist ca. doppelt so lang wie der Kontaktabschnitt 25 und erstreckt sich hier in Längsrichtung R über eine Strecke von 30 mm.

Wie ferner in Fig. 4 eingezeichnet ist, beträgt der Außendurchmesser der ersten Isolierung 39 1,8 mm.

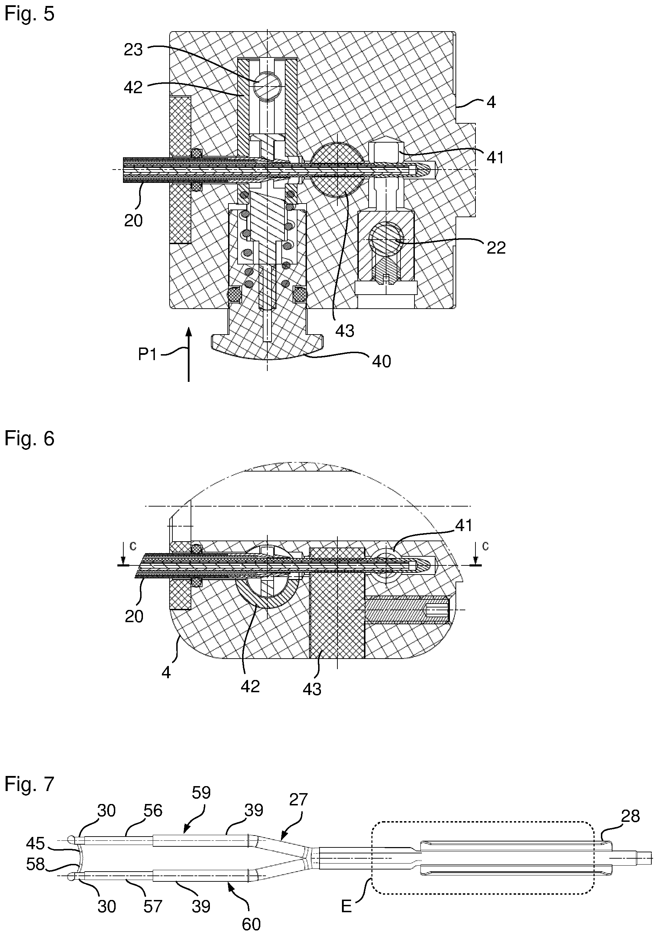

In Fig. 5 ist der Schnitt C-C (Fig. 3) durch den Führungsblock 4 mit eingesetzter Elektrode 20 und in Fig. 6 ist der Schnitt A-A (Fig. 3) durch den Führungsblock 4 mit eingesetzter Elektrode 20 gezeigt.

Im Führungsblock 4 ist ein federvorgespanntes Rastelement 40, das in Eingriff mit der Rasthülse 36 steht und somit die Elektrode 20 arretiert, wobei in diesem Zustand die Endhülse 30 in Kontakt mit einem ersten Kontaktabschnitt 41 steht, der die gewünschte elektrische Kontaktierung mit dem ersten elektrischen Anschluss 22 sicherstellt. Über einen zweiten Kontaktabschnitt 42 im Führungsblock 4 erfolgt die gewünschte elektrische Kontaktierung der Rasthülse 36 mit dem zweiten elektrischen Anschluss 23.

Zwischen den beiden Kontaktabschnitten 41 und 42 ist eine Dichtung 43 angeordnet, die zwischen Endhülse 30 und Rasthülse 36 an der zweiten Isolierung 35 anliegt. Mit dieser Dichtung 43 wird sichergestellt, dass kein elektrischer Kontakt zwischen Rasthülse 36 und Endhülse 30 vorliegt, obwohl Feuchtigkeit in diesem Bereich auftreten kann. Mit dieser Dichtung 43 wird also die gewünschte Potentialtrennung sichergestellt.

Um die Elektrode 20 aus dem Führungsblock 4 entnehmen zu können, muss lediglich das Rastelement 40 gegen die Federkraft in Richtung des Pfeiles P1 gedrückt werden, so dass die Arretierung gelöst wird und die Elektrode 20 entnommen werden kann. Für ein Einsetzen ist es lediglich notwendig, die Elektrode 20 so lange in den Führungsblock 4 einzuführen, bis das Rastelement 40 einrastet.

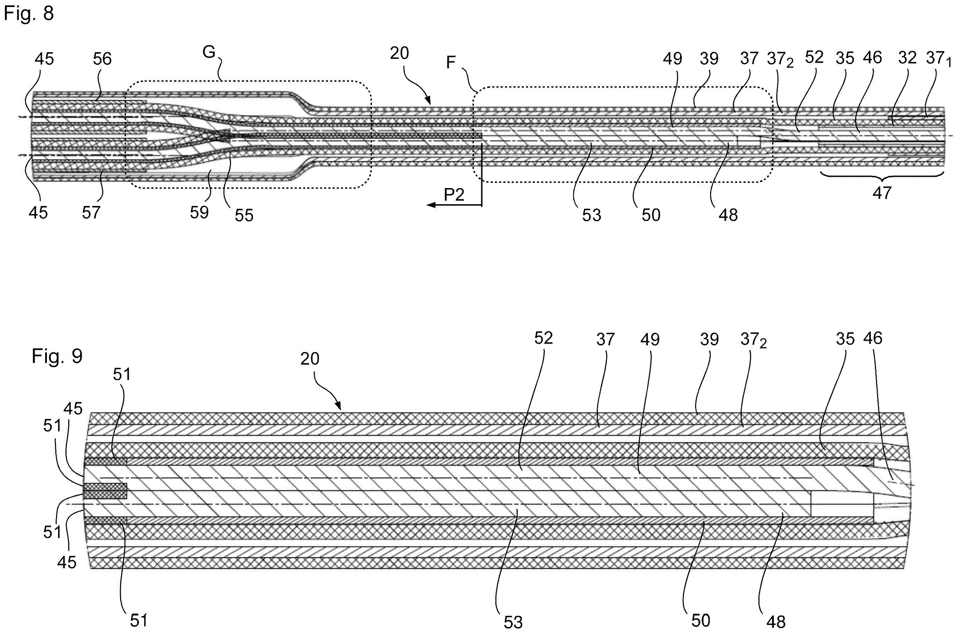

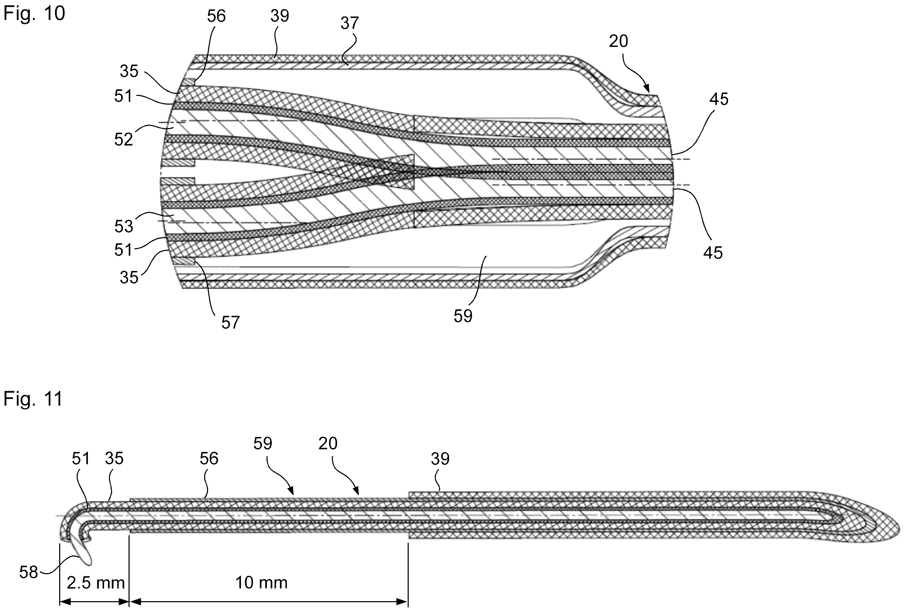

In der in Fig. 7 gezeigten Aufsicht des distalen Endabschnitts 27 ist deutlich die gabelförmige Ausbildung des distalen Endabschnitts 27 mit zwei Gabelarmen 59, 60 und einer Schlinge 58 eines Schlingendrahtes 45 dargestellt. Eine Schnittansicht entlang der Schnittlinie C-C (Fig. 3) des Bereiches E (Fig. 7) des Endabschnitts 27 ist in Fig. 8 gezeigt, wobei zur Vereinfachung der Darstellung die Führung 28 nicht gezeigt ist. Das Detail F von Fig. 8 ist in Fig. 9 und das Detail G von Fig. 8 ist in Fig. 10 gezeigt.

Ein erster proximaler Endabschnitt 46 des Schlingendrahts 45 ist im Bereich 47 mit dem Innenrohr 32 verpresst. Ein zweiter proximaler Endabschnitt 48 endet in axialer Richtung vor dem Bereich 47, so dass zwischen dem zweiten proximalen Endabschnitt 48 und dem Bereich 47 ein axialer Abstand vorhanden ist. Der zweite proximale Endabschnitt 48 ist mit einem vom ersten proximalen Endabschnitt 46 kommenden Abschnitt 49 des Schlingendrahts 45 mittels einer den zweiten proximalen Endabschnitt 48 und den Abschnitt 49 umgebenden Verbindungshülse 50 verpresst. In der hier beschriebenen Ausführungsform weist die Verbindungshülse 50 eine axiale Länge von 10 mm auf. Die beiden Teile des Schlingendrahtes 45, die mittels der Verbindungshülse 50 verpresst sind und bis zum distalen Ende 21 und somit bis zur Schlinge 58 laufen, werden nachfolgend erster und zweiter Drahtabschnitt 52, 53 genannt.

Ferner ist das Hauptrohr 37 zweiteilig ausgebildet, wobei der im Bereich 47 endende erste Teil 37₁ des Hauptrohrs 37 mit einen zweiten Teil 37₂ des Hauptrohrs 37 verpresst ist.

In axialer Richtung anschließend an die Verbindungshülse 50 (durch den Pfeil P2 angedeutet) ist eine dritte Isolierung 51 direkt auf die beiden Drahtabschnitten 52, 53 des Schlingendrahts 45 aufgebracht, die wiederum als Schrumpfschlauch (beispielsweise PTFE-Schlauch oder PVDF-Schlauch) ausgebildet sein kann. Diese dritte Isolierung 51 umgibt ab einem Gabelpunkt 55, an dem die beiden Drahtabschnitte 52, 53 auseinanderlaufen und somit in radialer Richtung voneinander beabstandet sind, jeden der beiden Schlingendrahtabschnitte 52, 53. Somit überlappt die zweite Isolierung 35 in axialer Richtung anschließend an die Verbindungshülse 50 die dritte Isolierung 51, wodurch eventuelle Kurzschlüsse zwischen Aktivelektrode 34 und Neutralelektrode 38 verhindert werden können, da in dem Bereich 59 um den Gabelpunkt 55 zwischen Hauptrohr 37 und Innenrohr 32 Flüssigkeit eindringen kann. Nach dem Gabelpunkt 55 in axialer Richtung zum distalen Ende 21 der Elektrode 20 hin ist um jeden Drahtabschnitt 52, 53 ein (erstes und zweites) Gabelrohr 56 und 57 ausgebildet, wobei das Hauptrohr 37 mit jedem Gabelrohr 56 und 57 verschweißt ist und dann etwas links vom Ende der Darstellung in Fig. 8 endet. Die erste Isolierung 39 läuft jedoch weiter und endet dann, wie insbesondere in der Schnittansicht D-D (Fig. 3) des Schlingendrahtabschnitts 52 in Fig. 11 sowie in Fig. 7 zu sehen ist, etwa 12,5 mm vor dem distalen Ende 21 der Elektrode 20. Das erste und zweite Gabelrohr 56, 57 liegen somit über einer Länge von 10 mm frei und bilden das distale Ende der Neutralelektrode 38 bzw. einen freiliegenden zweiten Endbereich der Neutralelektrode 38. Der Schlingendraht 45 liegt dann in dem die beiden Gabelarme 59, 60 verbindenden U-förmigen Bereich frei und bildet somit das distale Ende der Aktivelektrode 34 bzw. einen freiliegenden ersten Endbereich der Aktivelektrode 34. Der Schlingendraht 45 ist hier bevorzugt aus Wolfram gebildet.

Die Schlinge 58 ist nur ein Beispiel eines distalen Arbeitsendes 21 der bipolaren Elektrode 20. Das distale Ende 21 kann auch einen Kegel, ein Messer, eine Walze, eine Rolle, etc. aufweisen. Aufgrund der dann vorliegenden größeren Radien kann, muss aber nicht, statt Wolfram beispielsweise Edelstahl verwendet werden. Edelstahl kann dann auch für den gesamten Draht 45 verwendet werden.

Die Elektrode 20 kann somit als koaxiale bipolare Elektrode 20 bezeichnet werden, da die Neutralelektrode 38 koaxial zur Aktivelektrode 34 verläuft. Es werden ferner beide Elektroden 34 und 38 vom proximalen Kontaktabschnitt 25 bis zum distalen Ende 21 geführt, so dass im Betrieb der Stromfluss von der freiliegenden Schlinge 58 bis zum freiliegenden Endabschnitt des entsprechenden Gabelrohrs 56 bzw. 57 stattfindet. Somit muss in vorteilhafter Weise bei dieser Anwendung kein Strom über den Patienten zu- bzw. abgeführt werden und es liegt die volle Potentialdifferenz direkt an der Schlinge 58 und somit am distalen Ende 21 der bipolaren Elektrode 20 an. Dadurch kann z.B. ein gutes Zündverhalten für das gewünschte Plasma in einer NaCl-Lösung sichergestellt werden. Ferner ist in vorteilhafter Weise das Arbeitselement 2 selbst potentialfrei. Die Ausbildung des Innenrohrs 32 aus Kupfer ist vorteilhaft, da die auftretenden Stromstärken gut geleitet werden können, ohne dass Temperaturprobleme auftreten.

## Patentansprüche

1. Bipolare Elektrode für ein Resektoskop,
wobei die bipolare Elektrode (20) einen proximalen Kontaktabschnitt (25), einen daran anschließenden Mittelabschnitt (26) und einen daran anschließenden distalen Endabschnitt (27) aufweist,
wobei die bipolare Elektrode (20) eine Aktivelektrode (34) und eine Neutralelektrode (38) aufweist,
wobei die Neutralelektrode (38) im Mittelabschnitt (26) hohlzylinderförmig ausgebildet ist und die Aktivelektrode (34) koaxial umgibt,
wobei der proximale Kontaktabschnitt (25) einen ersten Kontakt (30) der Aktivelektrode (34) und einen zweiten Kontakt (36) der Neutralelektrode (38) aufweist.
wobei die Neutralelektrode (38) vom proximalen Kontaktabschnitt (25) bis zum distalen Endabschnitt (27) mit einer ersten Isolierung (39) umgeben ist,
und wobei am distalen Endabschnitt (27) sowohl ein erster Endbereich der Aktivelektrode (34) als auch ein zweiter Endbereich der Neutralelektrode (38) freiliegt, so dass eine über die Kontakte (30, 36) angelegte elektrische Spannung unmittelbar zwischen den beiden Endbereichen anliegt.

2. Bipolare Elektrode nach Anspruch 1,
wobei die Aktivelektrode (34) im Mittelabschnitt (26) einen Kupferleiter (32) aufweist.

3. Bipolare Elektrode nach Anspruch 1 oder 2,
wobei die Aktivelektrode (34) im Mittelabschnitt (26) einen hohlzylinderförmigen Leiter (32) aufweist.

4. Bipolare Elektrode nach Anspruch 3,
wobei im hohlzylinderförmigen Leiter (32) der Aktivelektrode (34) im Mittelabschnitt (26) ein leitfähiger Draht vorgesehen ist.

5. Bipolare Elektrode nach Anspruch 1 oder 2,
wobei die Aktivelektrode (34) im Mittelabschnitt (26) einen Vollmaterial-Leiter (32) aufweist.

6. Bipolare Elektrode nach einem der obigen Ansprüche,
wobei die Aktivelektrode (34) im Bereich des proximalen Kontaktabschnitts (25) ein Element (33) zur mechanischen Verstärkung aufweist.

7. Bipolare Elektrode nach einem der obigen Ansprüche,
wobei zwischen der Aktivelektrode (34) und der Neutralelektrode (38) eine zweite Isolierung (35) vorgesehen ist.

8. Bipolare Elektrode nach einem der obigen Ansprüche,
wobei der distalen Endabschnitt (27) gabelförmig mit einem ersten und einem zweiten Gabelarm (59, 60) ausgebildet ist, wobei der freiliegende erste Endbereich der Aktivelektrode (34) zwischen den beiden Gabelarmen (59, 60) liegt.

9. Bipolare Elektrode nach Anspruch 7 und 8,
wobei jeder Gabelarm (59, 60) eine dritte Isolierung aufweist, die zwischen der Aktivelektrode (34) und der Neutralelektrode (38) angeordnet ist und mit der zweiten Isolierung überlappt.

10. Bipolare Elektrode nach einem der obigen Ansprüche,
- wobei der freiliegende erste Endbereich der Aktivelektrode (34) als Wolframdraht (45) ausgebildet ist
und/oder
- wobei der erste Kontakt (30) der Aktivelektrode (34) und/oder der zweite Kontakt (36) der Neutralelektrode (38) eine metallische Hülse aufweist.

11. Bipolare Elektrode nach einem der obigen Ansprüche,
wobei der erste Kontakt (30) der Aktivelektrode (34) und/oder der zweite Kontakt (36) der Neutralelektrode (38) eine metallische Beschichtung aufweist.

12. Bipolare Elektrode nach einem der obigen Ansprüche,
wobei der zweite Kontakt (36) der Neutralelektrode (38) so ausgebildet ist, dass der zweite Kontakt (36) in Eingriff mit einer Rastverbindung gebracht werden kann.

13. Resektoskop mit einer bipolaren Elektrode nach einem der obigen Ansprüche.

14. Resektoskop nach Anspruch 13,
wobei das Resektoskop (1) einen Führungsblock (4) mit einem Rastelement (40) aufweist, das im eingesetzten Zustand der bipolaren Elektrode (20) eine Rastverbindung mit dem ersten Kontakt (30) der Aktivelektrode (34) oder dem zweiten Kontakt (30) der Neutralelektrode (36) bewirkt.

15. Resektoskop nach Anspruch 13 oder 14,
wobei das Resektoskop (1) einen Führungsblock (4) mit einem ersten Kontaktabschnitt (41) für den ersten Kontakt (30) der Aktivelektrode (34) und einem zweiten Kontaktabschnitt (42) für den zweiten Kontakt (36) der Neutralelektrode (38) aufweist, wobei zwischen beiden Kontaktabschnitten (41, 42) eine Dichtung (43) angeordnet ist, die im eingesetzten Zustand der bipolaren Elektrode (20) verhindert, dass eine Flüssigkeit entlang des proximalen Kontaktabschnitts (26) vom zweiten Kontakt (30) zum ersten Kontakt (36) gelangen kann.
